# EUROPEAN PATENT SPECIFICATION

(11) **EP 0 153 845 B1**
(45) Date of publication and mention of the grant of the patent: **21.04.1993**
(21) Application number: 85301093.2
(22) Date of filing: 19.02.1985
(51) Int. Cl.: C07K 7/10, A61K 37/02

(54) **CRF analogs**
CRF-Analoga
Analogues de CRF

(30) Priority: 23.02.1984 US 583092; 14.05.1984 US 610110
(43) Date of publication of application: 04.09.1985
(60) Divisional application: 92115541.2
(73) Proprietor: THE SALK INSTITUTE FOR BIOLOGICAL STUDIES, La Jolla California 92037 (US)
(72) Inventor: Rivier, Catherine Laure, La Jolla California 92037 (US); Rivier, Jean Edouard Frederic, La Jolla California 92037 (US); Vale, Wylie Walker, La Jolla California 92037 (US); Brown, Marvin Ross, Del Mar California 92014 (US)
(74) Representative: Allard, Susan Joyce

(56) References cited:
- EP-A- 0 067 608
- EP-A- 0 122 798
- US-A- 4 385 050
- US-A- 4 415 558
- PEPTIDES, PROCEEDINGS OF THE EUROPEAN PEPTIDE SYMPOSIUM, 17th; 1982, pages 597-602; Walter de Gruyter & Co., Berlin DE; J. RIVIER et al.: "Solid phase synthesis of amunine (CRF), sauvegine and two urotensin I"
- SCIENCE, vol. 218, no. 4568, 8th October 1982, pages 162-164, The Am. Ass. for the Advancement of Science, Washington, DC, US; K. DEDERIS et al.: "Complete amino acid sequence of urotensin I, a hyptensive and corticotropin-releasing neuropeptide from Catostomus"
- PROC. OF THE NATIONAL ACADEMY OF SCIENCES, vol. 80, August 1983, pages 4851-4855, Washington, DC, US; J. RIVIER et al.: "Characterization of rat hypothalamic corticotropin-realsing factor"
- BIOCHEMISTRY, vo. 22, 1983, pages 4341-4346, Am. Chem. Soc., US; J. SPIESS et al.: "Sequence analysis of rat hypothalamic corticotropin-releasing factor with the 0-phthalaldehyde strategy"
- EMBO-JOURNAL, vol. 2, no. 5, 1983, pages 775-779, IRL-Press, Oxford, GB; S. SHIBAHARA et al.: "Isolation oand sequence anlaysis of the human corticotropin-releasing factor precursor gene"
- CHEMICAL ABSTRACTS, vol. 102, 1985, page 634, abstract no. 204302j, Columbus, Ohio, US & JP-A-59 206 342 (MITSUBISHI CHEMICAL INDUSTRIES CO., LTD.) 22-11-1984
- SCIENCE, vol. 224, 25th May 1984, pages 889-891; Am. Ass. for the Advancement of Science, Washington, D.C. US; J. RIVIER et al.: "Synthetic competitive antagonists of corticotropin-reseasing factor: Effect on ACTH secretion int he rat"

## Description

This invention is directed to peptides and to methods for pharmaceutical treatment of mammals using such peptides. More specifically, the invention relates to the hentetracontapeptide CRF, to analogs of CRF, to pharmaceutical compositions containing CRF or such analogs and to methods of treatment of mammals using CRF or such analogs.

The hypothalamus plays a key role in the regulation of adenohypophysial corticotropic cells secretory functions. Factors in hypothalamus increase the rate of ACTH secretion by the pituitary gland. A physiologic corticotropin releasing factor (CRF), i.e., ovine CRF (oCRF), was characterized in 1981 and disclosed in U.S. Patent No. 4,415,558 to have the formula: 
H-Ser-Gln-Glu-Pro-Pro-Ile-Ser-Leu-Asp-Leu-Thr-Phe-His-Leu-
Leu-Arg-Glu-Val-Leu-Glu-Met-Thr-Lys-Ala-Asp-Gln-Leu-Ala-
Gln-Gln-Ala-His-Ser-Asn-Arg-Lys-Leu-Leu-Asp-Ile-Ala-NH2.
Rat CRF(rCRF) has been characterized as having
the formula: H-Ser-Glu-Glu-Pro-Pro-Ile-Ser-Leu-Asp-Leu-
Thr-Phe-His-Leu-Leu-Arg-Glu-Val-Leu-Glu-Met-Ala-Arg-Ala-
Glu-Gln-Leu-Ala-Gln-Gln-Ala-His-Ser-Asn-Arg-Lys-Leu-Met-
Glu-Ile-Ile-NH2
and may alternatively be referred to as rat Amunine. The formula of human CRF has apparently been determined to be the same as that of rCRF. Synthetic rCRF and oCRF stimulate ACTH and β-endorphin activites in vitro and in vivo and substantially lower blood pressure for an extended time period.

Analogs of these 41-residue CRF peptides, having the following formulae have at least substantially the same biological activity in the foregoing respects as the native peptides or are competitive antagonists of the natural peptides: 
H-Ser-Gln-Glu-Pro-Pro-Ile-Ser-Leu-Asp-Leu-Thr-Phe-His-
Leu-Leu-Arg-Glu-Val-Leu-Glu-Met-Thr-Arg-Ala-Asp-Gln-
Leu-Ala-Gln-Gln-Ala-His-Ser-Asn-Arg-Arg-Leu-Leu-Asp-
Ile-Ala-NH2,
or a nontoxic addition salt thereof, or a nontoxic addition salt thereof,
or 
H-D-Pro-Pro-Ile-Ser-Leu-Asp-Leu-Thr-Phe-His-Leu-Leu-
Arg-Glu-Val-Leu-Glu-Nle-Ala-Arg-Ala-Glu-Gln-Leu-Ala-
Gln-Gln-Ala-His-Ser-Asn-Arg-Lys-Leu-Nle-Glu-Ile-Ile-
NH2,
wherein the N-terminus can be lengthened by the addition of up to 3 of the residues, H-Ser-Gln-Glu, or of the residue D-Tyr, or a nontoxic addition salt thereof.
or
the compound having the formula: 
H-Leu-Asp-Leu-Thr-Phe-His-Leu-Leu-Arg-Glu-Val-Leu-Glu-
R21-R22-R23-Ala-R25-Gln-Leu-Ala-Gln-Gln-Ala-
His-Ser-Asn-Arg-R36-Leu-R38-R39-Ile-R41-NH2,
wherein R₂₁ is Met or Nle; R₂₂ is Ala or Thr; R₂₃ is Arg or Lys; R₂₅ is Asp or Glu; R₃₆ is Lys or Arg; R₃₈ is Met, Leu or Nle; R₃₉ is Glu or Asp; R₄₁ is Ala or Ile, or a nontoxic addition salt thereof.

In the third embodiment as described above, the group R₂₁ is preferably Nle and the group R₃₈ is preferably Nle. A particularly preferred compound is:

H-Leu-Asp-Leu-Thr-Phe-His-Leu-Leu-Arg-Glu-Val-
Leu-Glu-Met-Ala-Arg-Ala-Glu-Gln-Leu-Ala-Gln-Gln-Ala-His-
Ser-Asn-Arg-Lys-Leu-Met-Glu-Ile-Ile-NH2.

Pharmaceutical compositions in accordance with the invention include such CRF analogs, or nontoxic addition salts thereof, dispersed in a pharmaceutically or veterinarily acceptable liquid or solid carrier. The administration of such peptides or pharmaceutically or veterinarily acceptable addition salts thereof to mammals, particularly humans, in accordance with the invention may be carried out for the regulation of secretion of ACTH, β-endorphin, β-lipotropin, other products of the pro-opiomelanocortin gene and corticosterone and/or for the lowering of blood pressure and/or for affecting mood, behavioral and gastrointestinal functions and autonomic nervous system activities. Furthermore CRF analogs may be used for the evaluation of the status of pituitary, cardiovascular, gastrointestinal or central nervous system functions.

The nomenclature used to define the peptides is that specified by Schroder & Lubke, "The Peptides", Academic Press (1965) wherein, in accordance with conventional representation, the amino group appears to the left and the carboxyl group to the right. The standard 3-letter abbreviations to identify the alpha-amino acid residues, and where the amino acid residue has isomeric forms, it is the L-form of the amino acid that is represented unless otherwise expressly indicated, e.g. Ser = L-serine, Nle = L-norleucine, Nva = norvaline, Har = homoarginine, Orn = ornithine etc. In addition the following abbreviations are used: leu = either L-leucine or C∝CH₃-L-leucine (CML) and ala = either L-alanine or C∝CH₃-L-alanine(CMA).

The peptides are synthesized by a suitable method, such as by exclusively solid-phase techniques, by partial solid-phase techniques, by fragment condensation or by classical solution addition. Certain CRF analogs which do not include D-isomer residues or unnatural amino acid residues may also be synthesized by recently developed recombinant DNA techniques.

Synthesis by the use of recombinant DNA techniques, for purposes of this application, should be understood to include the suitable employment of a structural gene coding for the desired form of CRF analog. The synthetic CRF peptide may be obtained by transforming a microorganism using an expression vector including a promoter and operator together with such structural gene and causing such transformed microorganism to express the CRF peptide. A non-human animal may also be used to produce the CRF peptide by gene-farming using such a structural gene and the general techniques set forth in U.S. Patent No. 4,276,282 issued June 30, 1981 or using microinjection of embryos as described in W083/01783 published 26 May 1983 and W082/04443 published 23 December 1982. The synthetic CRF peptide is then suitably recovered from the animal by extraction from sera or the like.

Common to chemical syntheses of peptides is the protection of the labile side chain groups of the various amino acid moieties with suitable protecting groups which will prevent a chemical reaction from occurring at that site until the group is ultimately removed. Usually also common is the protection of an alpha-amino group on an amino acid or a fragment while that entity reacts at the carboxyl group, followed by the selective removal of the alpha-amino protecting group to allow subsequent reaction to take place at that location. Accordingly, it is common that, as a step in the synthesis, an intermediate compound is produced which includes each of the amino acid residues located in its desired sequence in the peptide chain with various of these residues having side-chain protecting groups.

Also considered to be within the scope of the present invention are the peptides as defined above which contain at least one protecting group of the formula X, X¹, X², X³, X⁴, X⁵, X⁶ or X⁷ which are defined as follows:
X¹ is an α-amino
protecting group. The ∝-amino protecting groups contemplated by X¹ are those known to be useful in the art in the step-wise synthesis of polypeptides. Among the classes of ∝-amino protecting groups covered by X¹ are (1) acyl-type protecting groups, such as formyl, acrylyl(Acr), benzoyl(Bz) and acetyl(Ac) which are preferably used only at the N-terminal; (2) aromatic urethan-type protecting groups, such as benzyloxycarbonyl(Z) and substituted Z, such as p-chlorobenzyloxycarbonyl, p-nitrobenzyloxycarbonyl, p-bromobenzyloxycarbonyl, p-methoxybenzyloxycarbonyl; (3) aliphatic urethan protecting groups, such as t-butyloxycarbonyl (BOC), diisopropylmethoxycarbonyl, isopropyloxycarbonyl, ethoxycarbonyl, allyloxycarbonyl; (4) cycloalkyl urethan-type protecting groups, such as fluorenylmethyloxycarbonyl(FMOC), cyclopentyloxycarbonyl, adamantyloxycarbonyl,and cyclohexyloxycarbonyl; and (5) thiourethan-type protecting groups, such as phenylthiocarbonyl. The preferred ∝-amino protecting group is BOC.

X² is a protecting group for the hydroxyl group of Thr and Ser and is preferably selected from the class consisting of acetyl(Ac), benzoyl(Bz), tert-butyl, triphenylmethyl(trityl), tetrahydropyranyl, benzyl ether(Bzl) and 2,6-dichlorobenzyl (DCB). The most preferred protecting group is Bzl. X² can be hydrogen, which means there is no protecting group on the hydroxyl group.

X³ is a protecting group for the guanidino group of Arg preferably selected from the class consisting of nitro, p-toluenesulfonyl(Tos), Z, adamantyloxycarbonyl and BOC, or is hydrogen. Tos is most preferred.

X⁴ is a protecting group, preferably xanthyl(Xan), for the amido group of Asn or Gln.

X⁵ is an ester-forming protecting group for the β- or γ-carboxyl group of Asp or Glu, preferably selected from the class consisting of benzyl, 2,6-dichlorobenzyl, methyl, ethyl and t-butyl ester. OBzl is most preferred.

X⁶ is a protecting group for the side chain amino substituent of Lys. Illustrative of suitable side chain amino protecting groups are Z, 2-chlorobenzyloxycarbonyl(2-Cl-Z), Tos, t-amyloxycarbonyl(Aoc), BOC and aromatic or aliphatic urethan-type protecting groups as specified hereinbefore.

For His, X is a protecting group for the imidazole nitrogen such as Tos or 2,4-dinitrophenyl(DNP), and for Tyr, X is a protecting group for the hydroxyl group such as DCB.

The selection of a side chain amino protecting group is not critical except that it should must be one which is not removed during deprotection of the ∝-amino groups during the synthesis. Hence, the ∝-amino protecting group and the side chain amino protecting group cannot be the same.

The NH₂ terminal group may be protected by a protecting group such as an ester or converted into an anchoring bond used in solid phase synthesis for linking to a solid resin support, preferably one represented by the formulae:
-NH-benzhydrylamine (BHA) resin support and -NH-paramethylbenzhydrylamine (MBHA) resin support. Cleavage from a BHA or MBHA resin directly gives the CRF analog amide. By employing a methyl-derivative of such a resin, a methyl-substituted amide can be created.

The intermediate containing at least one of X, X¹, X², X³ X⁴, X⁵ and X⁶ as a protecting group. The particular amino acid chosen for each the R-group determines whether there will also be a protecting group attached as specified hereinbefore and as generally known in the art. In selecting a particular side chain protecting group to be used in the synthesis of the peptides, the following rules are followed: (a) the protecting group should be stable to the reagent and under the reaction conditions selected for removing the ∝-amino protecting group at each step of the synthesis, (b) the protecting group should retain its protecting properties and not be split off under coupling conditions and (c) the side chain protecting group must be removable, upon the completion of the synthesis containing the desired amino acid sequence, under reaction conditions that will not alter the peptide chain.

Thus, the present invention is also considered to provide a process for the manufacture of the compounds of the invention comprising (a) forming an appropriate peptide containing at least one protecting group of the formula: X, X¹, X², X³, X⁴, X⁵, or X⁶ as defined above, and the terminal -NH₂ group is optionally protected or converted into an anchoring bond to resin support and (b) splitting off the protective group or groups or anchoring bond from the said peptide and (c) if desired, converting a resulting peptide into a nontoxic addition salt thereof.

When the peptides are prepared by chemical synthesis, they are preferably prepared using solid phase synthesis, such as that described by Merrifield, J. Am. Chem. Soc., 85, p 2149 (1964), although other equivalent chemical syntheses known in the art can also be used as previously mentioned. Solid-phase synthesis is commenced from the C-terminal end of the peptide by coupling a protected ∝-amino acid to a suitable resin as generally set forth in U.S. Patent No. 4,244,946 issued Jan. 21, 1981 to Rivier et al., the disclosure of which is incorporated herein by reference. Such a starting material for rCRF analogs can be prepared by attaching ∝-amino-protected Ile to a BHA resin.

Ile protected by BOC is coupled to the BHA resin using methylene chloride and dimethylformamide (DMF). Following the coupling of BOC-Ile to the resin support, the ∝-amino protecting group is removed, as by using trifluoroacetic acid(TFA) in methylene chloride, TFA alone or with HCl in dioxane. Preferably 50 volume % TFA in methylene chloride is used with 0-5 weight % 1,2 ethanedithiol. The deprotection is carried out at a temperature between about 0°C and room temperature. Other standard cleaving reagents and conditions for removal of specific ∝-amino protecting groups may be used as described in Schroder & Lubke, "The Peptides", 1 pp 72-75 (Academic Press 1965).

After removal of the ∝-amino protecting group of Ile, the remaining ∝-amino- and side chain-protected amino acids are coupled step-wise in the desired order to obtain the intermediate compound defined hereinbefore. As an alternative to adding each amino acid separately in the synthesis, some of them may be coupled to one another prior to addition to the solid phase reactor. The selection of an appropriate coupling reagent is within the skill of the art. Particularly suitable as coupling reagents are N,N'-dicyclohexyl carbodiimide(DCCI) and N,N'-diisopropyl carbodiimide (DICI).

The activating reagents used in the solid phase synthesis of the peptides are well known in the peptide art. Examples of suitable activating reagents are carbodiimides, such as N,N'-diisopropyl carbodiimide and N-ethyl-N'-(3-dimethylaminopropyl)carbodiimide. Other activating reagents and their use in peptide coupling are described by Schroder & Lubke, supra, in Chapter III and by Kapoor, J. Phar. Sci., 59, pp 1-27 (1970).

Each protected amino acid or amino acid sequence is introduced into the solid phase reactor in about a fourfold excess, and the coupling is carried out in a medium of dimethylformamide(DMF):CH₂Cl₂ (1:1) or in DMF or CH₂Cl₂ alone. In instances where the coupling is carried out manually, the success of the coupling reaction at each stage of the synthesis is monitored by the ninhydrin reaction, as described by E. Kaiser et al., Anal. Biochem. 34, 595 (1970). In cases where incomplete coupling occurs, the coupling procedure is repeated before removal of the ∝-amino protecting group prior to the coupling of the next amino acid. The coupling reactions can be performed automatically, as on a Beckman 990 automatic synthesizer, using a program such as that reported in Rivier et al., Biopolymers, 1978, 17, pp.1927-1938.

After the desired amino acid sequence has been completed, the intermediate peptide is removed from the resin support by treatment with a reagent, such as liquid hydrogen fluoride, which not only cleaves the peptide from the resin but also cleaves all remaining side chain protecting groups X², X³, X⁴, X⁵ and X⁶ and the ∝-amino protecting group X¹ (unless it is an acyl group which is intended to be present in the final peptide), to obtain the peptide. When using hydrogen fluoride for cleaving, anisole or cresole and methylethyl sulfide are included in the reaction vessel as scavengers. When Met is present in the sequence, the BOC protecting group may be cleaved with trifluoroacetic acid(TFA)/ethanedithiol prior to cleaving the peptide from the resin to eliminate S-alkylation.

The following Example sets forth the preferred method for synthesizing CRF analogs by the solid-phase technique.

### EXAMPLE I (not of the invention)

The synthesis of [Glu²⁹]-rCRF having the formula: 
H-Ser-Glu-Glu-Pro-Pro-Ile-Ser-Leu-Asp-Leu-Thr-
Phe-His-Leu-Leu-Arg-Glu-Val-Leu-Glu-Met-Ala-Arg-Ala-Glu-
Gln-Leu-Ala-Glu-Gln-Ala-His-Ser-Asn-Arg-Lys-Leu-Met-Glu-
Ile-Ile-NH2
is conducted in a stepwise manner on a MBHA hydrochloride resin, such as available from Bachem, Inc., having a substitution range of about 0.1 to 0.5 mmoles/gm. resin.
The synthesis is performed on an automatic Beckman 990B peptide synthesizer using a suitable program, preferably as follows:

| STEP | REAGENTS AND OPERATIONS | MIX TIMES MIN. |
|---|---|---|
| 1 | CH₂Cl₂ wash-80 ml. (2 times) | 3 |
| 2 | Methanol(MeOH) wash-30 ml. (2 times) | 3 |
| 3 | CH₂Cl₂ wash-80 ml. (3 times) | 3 |
| 4 | 50 percent TFA plus 5 percent 1,2-ethane-dithiol in CH₂Cl₂-70 ml. (2 times) | 12 |
| 5 | Isopropanol wash-80 ml. (2 times) | 3 |
| 6 | TEA 12.5 percent in CH₂Cl₂-70 ml. (2 times) | 5 |
| 7 | MeOH wash-40 ml. (2 times) | 2 |
| 8 | CH₂Cl₂ wash-80 ml. (3 times) | 3 |
| 9 | Boc-amino acid (10 mmoles) in 30 ml. of either DMF or CH₂Cl₂, depending upon the solubility of the particular protected amino acid, (1 time) plus DCCI (10 mmoles) in CH₂Cl₂ | 30-300 |

Coupling of BOC-Ile results in the substitution of about 0.35 mmol. Ile per gram of resin. All solvents that are used are carefully degassed, preferably by sparging with an inert gas, e.g. helium or nitrogen, to insure the absence of oxygen that might undesirably oxidize the sulfur of the Met residue.

After deprotection and neutralization, the peptide chain is built step-by-step on the resin. Generally, one to two mmol. of BOC-protected amino acid in methylene chloride is used per gram of resin, plus one equivalent of 2 molar DCCI in methylene chloride, for two hours. When BOC-Arg(Tos) is being coupled, a mixture of 50% DMF and methylene chloride is used. Bzl is used as the hydroxyl side-chain protecting group for Ser and Thr. P-nitrophenyl ester(ONp) is used to activate the carboxyl end of Asn or Gln, and for example, BOC-Asn(ONp) is coupled overnight using one equivalent of HOBt in a 50% mixture of DMF and methylene chloride. The amido group of Asn or Gln is protected by Xan when DCCI coupling is used instead of the active ester method. 2-Cl-2 is used as the protecting group for the Lys side chain. Tos is used to protect the guanidino group of Arg and the imidazole group of His, and the side chain carboxyl group of Glu or Asp is protected by OBzl. At the end of the synthesis, the following
composition is obtained 
BOC-Ser(Bzl)-Glu(OBzl)-
Glu(OBzl)-Pro-Pro-Ile-Ser(Bzl)-Leu-Asp(OBzl)-Leu-Thr(Bzl)-
Phe-His(Tos)-Leu-Leu-Arg(Tos)-Glu(OBzl)-Val-Leu-Glu(OBzl)-
Met-Ala-Arg(Tos)-Ala-Glu(OBzl)-Gln(Xan)-Leu-Ala-Glu(OBzl)-
Gln(Xan)-Ala-His(Tos)-Ser(Bzl)-Asn(Xan)-Arg(Tos)-Lys
(2-Cl-Z)-Leu-Met-Glu(OBzl)-Ile-Ile-resin support.
Xan may have been partially or totally removed by TFA treatment used to deblock the ∝-amino protecting group.

In order to cleave and deprotect the resulting protected peptide-resin, it is treated with 1.5 ml. anisole, 0.5 ml. of methylethylsulfide and 15 ml. hydrogen fluoride (HF) per gram of peptide-resin, first at -20°C. for 20 min. and then at 0.°C. for one-half hour. After elimination of the HF under high vacuum, the resin-peptide is washed alternately with dry diethyl ether and chloroform, and the peptides are then extracted with de-gassed 2N aqueous acetic acid and separated from the resin by filtration.

The peptide is purified by gel permeation followed by semi-preparative HPLC as described in Rivier et al., Peptides: Structure and Biological Function (1979) pp. 125-128, and Rivier et al., J. Chromatography (1983). The chromatographic fractions are carefully monitored by HPLC, and only the fractions showing substantial purity were pooled.

To check whether the precise sequence was achieved, the rCRF analog was hydrolyzed in sealed evacuated tubes containing constant boiling HCl, 3µl of thioglycol/ml. and 1 nmol of Nle (as an internal standard) for 9 hours at 140°C. Amino acid analyses of the hydrolysates using a Beckman 121 MB amino acid analyzer showed the following amino acid ratios: Asx(1.9), Thr(0.8), Ser (3.1), Glx(9.0), Pro (2.1), Ala (3.8), Val(0.9), Met(1.9), Ile(2.6), Leu(7.0), Phe(0.9), Lys(1.0), His(2.0) and Arg(3.0), which confirmed that the 41-residue peptide structure had been obtained.

### EXAMPLE II

The synthetic peptide AHC and oCRF were examined for their effects on the secretion of ACTH and β-endorphin in vitro and was also in vivo. The potency of synthetic oCRF to stimulate the secretion of ACTH and β-endorphin by cultured rat pituitary cells was measured using the procedure as generally set forth in Endocrinology, 91, 562 (1972). Half-maximal responses were observed at about 65 picomolar concentrations of the peptide AHC, while synthetic oCRF concentrations of about 250 picomolar were needed to achieve this response. The secretory response to maximal (1-5 nM) concentrations of AHC is at a plateau level. In vivo testing is carried out using the general procedure set forth in C. Rivier et al., Science, 218, 377 (1982).

### EXAMPLE III

The peptide [Arg^{23,36}]-oCRF having the formula: 
H-Ser-Gln-Glu-Pro-Pro-Ile-Ser-Leu-Asp-Leu-Thr-Phe-His-Leu-
Leu-Arg-Glu-Val-Leu-Glu-Met-Thr-Arg-Ala-Asp-Gln-Leu-Ala-
Gln-Gln-Ala-His-Ser-Asn-Arg-Arg-Leu-Leu-Asp-Ile-Ala-NH2
is synthesized. Testing in accordance with the general procedure set forth in Example II shows that it stimulates the secretion of ACTH and β-END-LI to an amount equal to about 370% of oCRF and also causes a very significant lowering of blood pressure.

### EXAMPLE IV

The peptide [D-Pro⁴, Nle^{21,38}]-rCRF(4-41) having the formula: 
H-D-Pro-Pro-Ile-Ser-Leu-Asp-Leu-Thr-Phe-His-Leu-Leu-Arg-
Glu-Val-Leu-Glu-Nle-Ala-Arg-Ala-Glu-Gln-Leu-Ala-Gln-Gln-
Ala-His-Ser-Asn-Arg-Lys-Leu-Met-Glu-Ile-Ile-NH2
is synthesized. Testing in accordance with the general procedure set forth in Example II shows that it stimulates the secretion of ACTH and β-END-LI in an amount greater than oCRF and also causes a very significant lowering of blood pressure.

### EXAMPLE V

The peptide hCRF(8-41) having the formula: 
H-Leu-Asp-Leu-Thr-Phe-His-Leu-Leu-Arg-Glu-Val-Leu-Glu-
Met-Ala-Arg-Ala-Glu-Gln-Leu-Ala-Gln-Gln-Ala-His-Ser-Asn-
Arg-Lys-Leu-Met-Glu-Ile-Ile-NH2
is synthesized.

This synthetic CRF antagonist was examined for its effect on the secretion of ACTH and β-endorphin in vitro and the synthetic AHC peptide is also examined in vivo. The effectiveness of synthetic CRF antagonists to block the secretion of ACTH and β-endorphin by cultured rat pituitary cells is measured using the procedure as generally set forth in Vale et al., Endocrinology, 91, 562 (1972). In vivo testing is carried out using the general procedure set forth in C. Rivier et al., Science, 218, 377 (1982).

Based upon five independent experiments what we herein term the standard antagonist, AHC (9-41), blocks the secretion of ACTH due to 1 nMoCRF by 50%, at a concentration of 197 ± 72 nM. The specificity of this inhibition is demonstrated by the finding of no effect of the standard antagonist on the GRF-stimulated secretion of GH, the GnRH-stimulated secretion of LH and FSH or the TRF-stimulated secretion of TSH and prolactin. The effects of the antagonist on a number of different concentrations of oCRF and the ability of several different concentrations of AHC (9-41) to inhibit ACTH secretion stimulated by a constant dose of oGRF (1 nM) are considered to demonstrate competitive inhibition.

The in vivo effect of CRF antagonists is tested on the spontaneous ACTH release by adrenalectomized rats. The iv injection of 3 mg/kg BW (2.7 nmole) causes a marked decrease in plasma ACTH levels (measured as described in Vale et al. Science, 213, 1394, 1981), which is statistically significant for 2 hours. In the intact, non-anesthetized rats, the antagonist induces a dose-related inhibition of CRF-induced ACTH secretion, which is significant at the 0.09 µmole dose level. The antagonist AHC (9-41) also prevents most, but not all, of the ACTH rise due to ether-exposure.

These results indicate that administration of CRF antagonists reduces the spontaneous ACTH release observed after removal of the corticosteroid feedback, totally blocks the ACTH secretion caused by CRF, and inhibits most of the stressor-induced ACTH release in intact rats. Such data are comparable to those previously obtained in our laboratory with an antiserum to CRF which demonstrate the role played by endogenous CRF in regulating ACTH secretion, Rivier, C. et al., Science, 218, 377-9(1982).

These results confirm the hypothesis that CRF is indeed a critical element in the regulation of ACTH under several circumstances. In addition, that CRF antagonists can partially block the ether-exposure-induced activation of the sympathetic nervous system suggest a much broader role for this neuropeptide in mediating the response to stressful stimuli.

Synthetic hCRF has been shown to be a powerful stimulator of ACTH and β-END-LI secretion in vivo in several rat preparations. Plasma levels of ACTH and β-END-LI are elevated for at least 5-20 minutes following the intraveneous administration of hCRF to nembutal-anesthesized male rats and to quiescent male or female rats with indwelling intravenous cannulae. In addition, hCRF is found to have a dramatic effect to lower blood pressure in rats and dogs.

### EXAMPLE VI

The peptide [Lys²³, Leu³⁸]-hCRF(8-41) having the formula: 
H-Leu-Asp-Leu-Thr-Phe-His-Leu-Leu-
Arg-Glu-Val-Leu-Glu-Met-Ala-Lys-Ala-Glu-Gln-Leu-Ala-
Gln-Gln-Ala-His-Ser-Asn-Arg-Lys-Leu-Leu-Glu-Ile-Ile-NH2
is synthesized. Testing in accordance with the general procedure set forth in Example V shows that it likewise inhibits the secretion of ACTH and β-END-LI.

CRF agonists exhibit such lowering of blood pressure that they may be particularly valuable for the treatment of high blood pressure conditions and also for the treatment of patients who are to undergo certain types of surgery.

CRF profoundly stimulates the pituitaryadrenalcortical axis, and CRF analogs should be useful to stimulate the functions of this axis in some types of patients with low endogenous glucocorticoid production. For example, CRF should be useful in restoring pituitary-adrenal function in patients having received exogenous glucocorticoid therapy whose pituitary-adrenalcortical functions remain supressed. For example, CRF antagonists may be useful in regulating pituitary-adrenal function in patients having pituitary Cushings disease or any CRF-sensitive tumor.

Most other regulatory peptides have been found to have effects upon the central nervous system and upon the gastrointestinal tract. Because ACTH and β-END secretion is the "sine qua non" of mammal's response to stress, it was not surprising that CRF has significant effects on the brain as a mediator of the body's stress response. Accordingly, CRF analogs delivered to the brain should also find application in modifying the mood, learning and behavior of normal and mentally disordered individuals. Because CRF agonists elevate the levels of ACTH, β-END, β-lipotropin, other pro-opiomelanocortin gene products and corticosterone, their administration can be used to induce their effects on the brain and its periphery to thereby influence memory, mood, pain appreciation, etc., and more specifically, alertness, depression and/or anxiety, whereas CRF antagonists could be used to achieve opposite effects. For example, when administered into the ventricles, CRF increases activity and improves learning performance in rats and thus CRF agonists may function as a natural stimulant. Furthermore, CRF antagonists in the brain could ameliorate stress-induced conditions to which endogenous CRF might contribute, including some types of hypertension, infertility, decreased libido, impotentcy and hyperglycemia, as well as to modulate the immune system, gastrointestinal tract and adrenalcortical growth and function.

CRF analogs should also be of use for increasing blood flow to the gastrointestinal tract of mammals, particularly humans and other mammals. All CRF related peptides have been shown to dialate the mesenteric vascular bed. CRF antagonists may also be of use for decreasing blood flow to the gastrointestinal tract of mammals, particularly humans. Also, oCRF inhibits gastric acid production, and CRF analogs are expected to also be effective in the treatment of gastric ulcers by reducing gastric acid production and/or inhibiting gastrointestinal functions in a mammal.

CRF analogs or the nontoxic addition salts thereof, combined with a pharmaceutically or veterinarily acceptable carrier to form a pharmaceutical composition, may be administered to mammals, including humans, either intravenously, subcutaneously, intramuscularly, percutaneously, e.g. intranasally, intracerebrospinally or orally. The peptides should be at least about 90% pure and preferably should have a purity of at least about 98%; however, lower purities are effective and may well be used with mammals other than humans. This purity means that the intended peptide constitutes the stated weight % of all like peptides and peptide fragments present. Administration to humans may be employed by a physician to lower blood pressure or to stimulate endogenous gluco-corticoid production. The required dosage will vary with the particular condition being treated, with the severity of the condition and with the duration of desired treatment.

These agonists may also be used to evaluate hypothalamic pituitary adrenal function in mammals with suspected endocrine or central nervous system pathology by suitable administration followed by monitoring body functions. For example, administration may be used as a diagnostic tool to evaluate the basis of Cushing's disease and affective disorders, such as depressive illness.

Such peptides are often administered in the form of pharmaceutically or veterinarily acceptable nontoxic salts, such as acid addition salts or metal complexes, e.g., with zinc, iron, calcium, barium, magnesium, aluminum or the like (which are considered as addition salts for purposes of this application). Illustrative of such acid addition salts are hydrochloride, hydrobromide, sulphate, phosphate, tannate, oxalate, fumarate, gluconate, alginate, maleate, acetate, citrate, benzoate, succinate, malate, ascorbate, tartrate and the like. If the active ingredient is to be administered in tablet form, the tablet may contain a binder, such as tragacanth, corn starch or gelatin; a disintegrating agent, such as alginic acid; and a lubricant, such as magnesium stearate. If administration in liquid form is desired, sweetening and/or flavoring may be used, and intravenous administration in isotonic saline, phosphate buffer solutions or the like may be effected.

The peptides should be administered under the guidance of a physician, and pharmaceutical compositions will usually contain the peptide in conjunction with a conventional, pharmaceutically or veterinarily-acceptable carrier. Usually, the dosage will be from about 1 to about 200 micrograms of the peptide per kilogram of the body weight of the host animal. In some instances, treatment of subjects with these peptides can be carried out in lieu of the administration of ACTH or corticosteroids, in such instances a dosage as low as about 10 ng/Kg of body weight may be employed. As used herein all temperatures are °C and all ratios are by volume. Percentages of liquid materials are also by volume.

## Claims (Claims for the following Contracting State(s): BE, CH, DE, FR, GB, IT, LI LU, NL, SE)

1. The compound having the formula: 
H-Ser-Gln-Glu-Pro-Pro-Ile-Ser-Leu-Asp-Leu-Thr-Phe-His-
Leu-Leu-Arg-Glu-Val-Leu-Glu-Met-Thr-Arg-Ala-Asp-Gln-
Leu-Ala-Gln-Gln-Ala-His-Ser-Asn-Arg-Arg-Leu-Leu-Asp-
Ile-Ala-NH2,
 or a nontoxic addition salt thereof.

2. The compound having the formula: 
H-D-Pro-Pro-Ile-Ser-Leu-Asp-Leu-Thr-Phe-His-Leu-Leu-
Arg-Glu-Val-Leu-Glu-Nle-Ala-Arg-Ala-Glu-Gln-Leu-Ala-
Gln-Gln-Ala-His-Ser-Asn-Arg-Lys-Leu-Nle-Glu-Ile-Ile-
NH2,
 wherein the N-terminus can be lengthened by the addition of up to 3 of the residues, H-Ser-Gln-Glu, or of the residue D-Tyr, or a nontoxic addition salt thereof.

3. The compound having the formula: 
H-Leu-Asp-Leu-Thr-Phe-His-Leu-Leu-Arg-Glu-Val-Leu-Glu-
R21-R22-R23-Ala-R25-Gln-Leu-Ala-Gln-Gln-Ala-
His-Ser-Asn-Arg-R36-Leu-R38-R39-Ile-R41-
NH2,
 wherein R₂₁ is Met or Nle; R₂₂ is Ala or Thr; R₂₃ is Arg or Lys; R₂₅ is Asp or Glu; R₃₆ is Lys or Arg; R₃₈ is Met, Leu or Nle; R₃₉ is Glu or Asp; R₄₁ is Ala or Ile, or a nontoxic addition salt thereof.

4. The compound of claim 3 wherein R₂₁ is Nle.

5. The compound of claim 3 or claim 4 wherein R₃₈ is Nle.

6. The compound of claim 3 having the formula:

H-Leu-Asp-Leu-Thr-Phe-His-Leu-Leu-Arg-Glu-Val-Leu-Glu-
Met-Ala-Arg-Ala-Glu-Gln-Leu-Ala-Gln-Gln-Ala-His-Ser-

Asn-Arg-Lys-Leu-Met-Glu-Ile-Ile-HN2.

7. A pharmaceutical or veterinary composition which comprises at least one synthetic peptide as claimed any one of claim 1 to 6 together with a pharmaceutically or veterinarily acceptable diluent or carrier therefor.

8. A composition for lowering stress in mammals which comprises an effective amount of a synthetic peptide or a nontoxic addition salt thereof having the amino acid sequence as specified in any one of claims 3 to 6 and a pharmaceutically or veterinarily acceptable liquid or solid carrier therefor.

## Claims (Claims for the following Contracting State(s): AT)

1. A method for the manufacture of compounds of one of the following formulae:
(i) 
H-Ser-Gln-Glu-Pro-Pro-Ile-Ser-Leu-Asp-Leu-
Thr-Phe-His-Leu-Leu-Arg-Glu-Val-Leu-Glu-
Met-Thr-Arg-Ala-Asp-Gln-Leu-Ala-Gln-Gln-
Ala-His-Ser-Asn-Arg-Arg-Leu-Leu-Asp-Ile-
Ala-NH2,
 or a nontoxic addition salt thereof.
(ii) 
H-D-Pro-Pro-Ile-Ser-Leu-Asp-Leu-Thr-Phe-
His-Leu-Leu-Arg-Glu-Val-Leu-Glu-Nle-Ala-
Arg-Ala-Glu-Gln-Leu-Ala-Gln-Gln-Ala-His-
Ser-Asn-Arg-Lys-Leu-Nle-Glu-Ile-Ile-NH2,
 wherein the N-terminus can be lengthened by the addition of up to 3 of the residues H-Ser-Gln-Glu, or of the residue D-Tyr; or
(iii) 
H-Leu-Asp-Leu-Thr-Phe-His-Leu-Leu-Arg-Glu-
Val-Leu-Glu- R21-R22-R23-Ala-R25-
Gln-Leu-Ala-Gln-Gln-Ala-His-Ser-Asn-Arg-
R36-Leu-R38-R39-Ile-R41-NH2,
 wherein R₂₁ is Met or Nle; R₂₂ is Ala or Thr; R₂₃ is Arg or Lys; R₂₅ is Asp or Glu; R₃₆ is Lys or Arg; R₃₈ is Met, Leu or Nle; R₃₉ is Glu or Asp; R₄₁ is Ala or Ile,
or a nontoxic addition salt thereof, which method comprises
(a) forming a peptide of one of the formulae (i), (ii) or (iii) having at least one protecting group and wherein the NH₂ terminal group may be unprotected, protected with a protecting group or bonded to a resin support,
(b) splitting of the protecting group or groups or anchoring bond from the peptide, and
(c) if desired, converting the resulting peptide into a nontoxic addition salt thereof.

2. A method as claimed in claim 1 wherein in formula (iii) R₂₁ is Nle.

3. A method as claimed in claim 1 wherein in formula (iii) R₃₈ is Nle.

4. A method as claimed in claim 1 wherein the compound of formula (iii) is

H-Leu-Asp-Leu-Thr-Phe-
His-Leu-Leu-Arg-Glu-Val-Leu-Glu-Met-Ala-Arg-Ala-Glu-
Gln-Leu-Ala-Gln-Gln-Ala-His-Ser-Asn-Arg-Lys-Leu-Met-
Glu-Ile-Ile-HN2.

5. A process for the preparation of a pharmaceutical or veterinary composition which comprises admixing at least one synthetic peptide as claimed in claim 1 with a pharmaceutically or veterinarily acceptable diluent or carrier therefor.

6. A process for the preparation of a composition for lowering stress in mammals which comprises admixing an effective amount of a synthetic peptide or a nontoxic addition salt thereof having the amino acid sequence as specified in formula (iii) of claim 1 or any one of claims 2 to 4 with a pharmaceutically or veterinarily acceptable liquid or solid carrier therefor.

## Patentansprüche (Patentansprüche für folgende(n) Vertragsstaat(en): BE, CH, DE, FR, GB, IT, LI, LU, NL, SE)

1. Verbindung mit der Formel: 
H-Ser-Gln-Glu-Pro-Pro-Ile-Ser-Leu-Asp-Leu-Thr-Phe-His-
Leu-Leu-Arg-Glu-Val-Leu-Glu-Met-Thr-Arg-Ala-Asp-Gln-Leu-
Ala-Gln-Gln-Ala-His-Ser-Asn-Arg-Arg-Leu-Leu-Asp-Ile-Ala-
NH2
 oder ein nicht-toxisches Additionssalz davon.

2. Verbindung mit der Formel: 
H-D-Pro-Pro-Ile-Ser-Leu-Asp-Leu-Thr-Phe-His-Leu-Leu-Arg-
Glu-Val-Leu-Glu-Nle-Ala-Arg-Ala-Glu-Gln-Leu-Ala-Gln-Gln-
Ala-His-Ser-Asn-Arg-Lys-Leu-Nle-Glu-Ile-Ile-NH2,
 worin der N-Terminus durch Anfügung von bis zu drei der Reste H-Ser-Gln-Glu oder des Rests D-Tyr verlängert werden kann, oder ein nicht-toxisches Additionssalz davon.

3. Verbindung mit der Formel: 
H-Leu-Asp-Leu-Thr-Phe-His-Leu-Leu-Arg-Glu-Val-Leu-Glu-
R21-R22-R23-Ala-R25-Gln-Leu-Ala-Gln-Gln-Ala-His-Ser-Asn-
Arg-R36-Leu-R38-R39-Ile-R41-NH2,
 worin R₂₁ Met oder Nle ist, R₂₂ Ala oder Thr ist, R₂₃ Arg oder Lys ist, R₂₅ Asp oder Glu ist, R₃₆ Lys oder Arg ist, R₃₈ Met, Leu oder Nle ist, R₃₉ Glu oder Asp ist, R₄₁ Ala oder Ile ist, oder ein nicht-toxisches Additionssalz davon.

4. Verbindung nach Anspruch 3, worin R₂₁ Nle ist.

5. Verbindung nach Anspruch 3 oder 4, worin R₃₈ Nle ist.

6. Verbindung nach Anspruch 3 mit der Formel:

H-Leu-Asp-Leu-Thr-Phe-His-Leu-Leu-Arg-Glu-Val-Leu-Glu-
Met-Ala-Arg-Ala-Glu-Gln-Leu-Ala-Gln-Gln-Ala-His-Ser-Asn-
Arg-Lys-Leu-Met-Glu-Ile-Ile-NH2.

7. Pharmazeutische oder veterinärmedizinische Zusammensetzung, die mindestens ein synthetisches Peptid nach einem der Ansprüche 1 bis 6 zusammen mit einem pharmazeutisch oder veterinärmedizinisch verträglichen Verdünnungsmittel oder Träger dafür enthält.

8. Zusammensetzung zur Verringerung von Streß in Säugern, umfassend eine wirksame Menge eines synthetischen Peptids oder eines nicht-toxischen Additionssalzes davon mit der Aminosäuresequenz gemäß einem der Ansprüche 3 bis 6 und einen pharmazeutisch oder veterinärmedizinisch verträglichen flüssigen oder festen Träger dafür.

## Patentansprüche (Patentansprüche für folgende(n) Vertragsstaat(en): AT)

1. Verfahren zur Herstellung von Verbindungen einer der folgenden Formeln:
(i) 
H-Ser-Gln-Glu-Pro-Pro-Ile-Ser-Leu-Asp-Leu-Thr-Phe-
His-Leu-Leu-Arg-Glu-Val-Leu-Glu-Met-Thr-Arg-Ala-
Asp-Gln-Leu-Ala-Gln-Gln-Ala-His-Ser-Asn-Arg-Arg-
Leu-Leu-Asp-Ile-Ala-NH2,
 oder einem nicht-toxischen Additionssalz davon,
(ii) 
H-D-Pro-Pro-Ile-Ser-Leu-Asp-Leu-Thr-Phe-His-Leu-
Leu-Arg-Glu-Val-Leu-Glu-Nle-Ala-Arg-Ala-Glu-Gln-
Leu-Ala-Gln-Gln-Ala-His-Ser-Asn-Arg-Lys-Leu-Nle-
Glu-Ile-Ile-NH2,
 worin der N-Terminus durch Anfügung von bis zu drei der Reste H-Ser-Gln-Glu oder des Rests D-Tyr verlängert werden kann, oder
(iii) 
H-Leu-Asp-Leu-Thr-Phe-His-Leu-Leu-Arg-Glu-Val-Leu-
Glu-R21-R22-R23-Ala-R25-Gln-Leu-Ala-Gln-Gln-Ala-
His-Ser-Asn-Arg-R36-Leu-R38-R39-Ile-R41-NH2,
 worin R₂₁ Met oder Nle ist, R₂₂ Ala oder Thr ist, R₂₃ Arg oder Lys ist, R₂₅ Asp oder Glu ist, R₃₆ Lys oder Arg ist, R₃₈ Met, Leu oder Nle ist, R₃₉ Glu oder Asp ist, R₄₁ Ala oder Ile ist,
oder einem nicht-toxischen Additionssalz davon, wobei das Verfahren umfaßt
(a) Herstellen eines Peptids einer der Formeln (i), (ii) oder (iii) mit mindestens einer Schutzgruppe und worin die NH₂-terminale Gruppe ungeschützt, mit einer Schutzgruppe geschützt oder an einen Harzträger gebunden sein kann,
(b) Abspalten der Schutzgruppe oder -gruppen oder Ankerbindung vom Peptid und
(c) sofern erwünscht, Überführen des resultierenden Peptids in ein nicht-toxisches Additionssalz davon.

2. Verfahren nach Anspruch 1, worin in Formel (iii) R₂₁ Nle ist.

3. Verfahren nach Anspruch 1, worin in Formel (iii) R₃₈ Nle ist.

4. Verfahren nach Anspruch 1, worin die Verbindung der Formel (iii) 
H-Leu-Asp-Leu-Thr-Phe-His-Leu-Leu-Arg-Glu-
Val-Leu-Glu-Met-Ala-Arg-Ala-Glu-Gln-Leu-Ala-Gln-Gln-Ala-
His-Ser-Asn-Arg-Lys-Leu-Met-Glu-Ile-Ile-NH2
 ist.

5. Verfahren zur Herstellung einer pharmazeutischen oder veterinärmedizinischen Zusammensetzung, umfassend das Vermischen von mindestens einem synthetischen Peptid nach Anspruch 1 mit einem pharmazeutisch oder veterinärmedizinisch verträglichen Verdünnungsmittel oder Träger dafür.

6. Verfahren zur Herstellung einer Zusammensetzung zur Verringerung von Streß in Säugern, umfassend das Vermischen einer wirksamen Menge eines synthetischen Peptids oder eines nicht-toxischen Additionssalzes davon mit der Aminosäuresequenz, wie in Formel (iii) von Anspruch 1 oder einem der Ansprüche 2 bis 4 spezifiziert, mit einem pharmazeutisch oder veterinärmedizinisch verträglichen flüssigen oder festen Trägern dafür.

## Revendications (Revendications pour l'(les) Etat(s) contractant(s) suivant(s): BE, CH, DE, FR, GB, IT, LI, LU, NL, SE)

1. Composé ayant pour formule : 
H-Ser-Gln-Glu-Pro-Pro-Ile-Ser-Leu-Asp-Leu-Thr-Phe-His-Leu-
Leu-Arg-Glu-Val-Leu-Glu-Met-Thr-Arg-Ala-Asp-Gln-Leu-Ala-Gln-
Gln-Ala-His-Ser-Asn-Arg-Arg-Leu-Leu-Asp-Ile-Ala-NH2,
 ou un sel non toxique de celui-ci obtenu par addition.

2. Composé ayant pour formule : 
H-D-Pro-Pro-Ile-Ser-Leu-Asp-Leu-Thr-Phe-His-Leu-Leu-Arg-Glu-
Val-Leu-Glu-Nle-Ala-Arg-Ala-Glu-Gln-Leu-Ala-Gln-Gln-Ala-His-
Ser-Asn-Arg-Lys-Leu-Nle-Glu-Ile-Ile-NH2,
 dans lequel l'extrémité N-terminale peut être allongée par l'addition de jusqu'à 3 résidus, H-Ser-Gln-Glu, ou par le résidu D-Tyr, ou un sel non toxique de celui-ci obtenu par addition.

3. Composé ayant pour formule : 
H-Leu-Asp-Leu-Thr-Phe-His-Leu-Leu-Arg-Glu-Val-Leu-Glu-R21-
R22-R23-Ala-R25-Gln-Leu-Ala-Gln-Gln-Ala-His-Ser-Asn-Arg-R36-
Leu-R38-R39-Ile-R41-NH2,
 dans lequel R₂₁ est Met ou Nle; R₂₂ est Ala ou Thr; R₂₃ est Arg ou Lys; R₂₅ est Asp ou Glu; R₃₆ est Lys ou Arg; R₃₈ est Met, Leu ou Nle; R₃₉ est Glu ou Asp; R₄₁ est Ala ou Ile, ou un sel non toxique correspondant obtenu par addition.

4. Composé de la revendication 3 dans lequel R₂₁ est Nle.

5. Composé de la revendication 3 ou de la revendication 4 dans lequel R₃₈ est Nle.

6. Composé de la revendication 3 ayant pour formule :

H-Leu-Asp-Leu-Thr-Phe-His-Leu-Leu-Arg-Glu-Val-Leu-Glu-Met-
Ala-Arg-Ala-Glu-Gln-Leu-Ala-Gln-Gln-Ala-His-Ser-Asn-Arg-Lys-
Leu-Met-Glu-Ile-Ile-HN2.

7. Composition pharmaceutique ou vétérinaire comprenant au moins l'un des peptides synthétiques revendiqués dans l'une quelconque des revendications 1 à 6 avec un diluant ou un véhicule acceptable du point de vue pharmaceutique ou vétérinaire de celui-ci.

8. Composition destinée à réduire le stress chez les mammifères comprenant une quantité efficace d'un peptide synthétique ou d'un sel de celui-ci obtenu par addition comme spécifié dans l'une quelconque des revendications 3 à 6 et un véhicule solide ou liquide acceptable du point de vue pharmaceutique ou vétérinaire pour celui-ci.

## Revendications (Revendications pour l'(les) Etat(s) contractant(s) suivant(s): AT)

1. Procédé de fabrication de composés ayant l'une des formules suivantes :
(i) 
H-Ser-Gln-Glu-Pro-Pro-Ile-Ser-Leu-Asp-Leu-Thr-Phe-His-
Leu-Leu-Arg-Glu-Val-Leu-Glu-Met-Thr-Arg-Ala-Asp-Gln-
Leu-Ala-Gln-Gln-Ala-His-Ser-Asn-Arg-Arg-Leu-Leu-Asp-
Ile-Ala-NH2,
 ou un sel non toxique correspondant obtenu par addition.
(ii) 
H-D-Pro-Pro-Ile-Ser-Leu-Asp-Leu-Thr-Phe-His-Leu-Leu-
Arg-Glu-Val-Leu-Glu-Nle-Ala-Arg-Ala-Glu-Gln-Leu-Ala-
Gln-Gln-Ala-His-Ser-Asn-Arg-Lys-Leu-Nle-Glu-Ile-Ile-
NH2,
 dans lequel l'extrémité N-terminale peut être allongée par l'addition de jusqu'à 3 résidus H-Ser-Gln-Glu, ou par le résidu D-Tyr; ou
(iii) 
H-Leu-Asp-Leu-Thr-Phe-His-Leu-Leu-Arg-Glu-Val-Leu-
Glu-R21-R22-R23-Ala-R25-Gln-Leu-Ala-Gln-Gln-Ala-His-
Ser-Asn-Arg-R36-Leu-R38-R39-Ile-R41-NH2,
 dans lequel R₂₁ est Met ou Nle; R₂₂ est Ala ou Thr; R₂₃ est Arg ou Lys; R₂₅ est Asp ou Glu; R₃₆ est Lys ou Arg; R₃₈ est Met, Leu ou Nle; R₃₉ est Glu ou Asp; R₄₁ est Ala ou Ile,
ou un sel non toxique correspondant obtenu par addition, ce procédé comprenant
(a) la formation d'un peptide ayant l'une des formules (i), (ii), ou (ii), possédant au moins un groupe protecteur et dans lequel groupement NH₂ terminal peut être non protégé, protégé par un groupe protecteur ou fixé sur un support de résine,
(b) le clivage du ou des groupe(s) protecteur(s) ou de la liaison fixant le peptide, et
(c) si souhaité, la conversion du peptide résultant en un sel non toxique de celui-ci par addition.

2. Procédé comme revendiqué dans la revendication 1 dans lequel R₂₁ de la formule (iii) est Nle.

3. Procédé comme revendiqué dans la revendication 1 dans lequel R₃₈ de la formule (iii) est Nle.

4. Procédé comme revendiqué dans la revendication 3 dans lequel le composé de formule (iii) est

H-Leu-Asp-Leu-Thr-Phe-His-
Leu-Leu-Arg-Glu-Val-Leu-Glu-Met-Ala-Arg-Ala-Glu-Gln-Leu-Ala-
Gln-Gln-Ala-His-Ser-Asn-Arg-Lys-Leu-Met-Glu-Ile-Ile-HN2.

5. Procédé pour la préparation d'une composition pharmaceutique ou vétérinaire comprenant le mélange d'au moins un des peptides synthétiques comme revendiqué dans la revendication 1 avec un diluant ou un véhicule acceptable du point de vue pharmaceutique ou vétérinaire de celui-ci.

6. Procédé pour la préparation d'une composition destinée à réduire le stress chez les mammifères comprenant le mélange d'une quantité efficace d'un peptide synthétique ou d'un sel de celui-ci obtenu par addition comme spécifié dans la formule (iii) de la revendication 1 ou l'une quelconque des revendications 2 à 4 avec un véhicule solide ou liquide acceptable du point de vue pharmaceutique ou vétérinaire pour celui-ci.
